# EUROPÄISCHE PATENTSCHRIFT

(11) **EP 1 218 326 B1**
(45) Veröffentlichungstag und Bekanntmachung des Hinweises auf die Patenterteilung: **03.11.2004**
(21) Anmeldenummer: 00960507.2
(22) Anmeldetag: 22.08.2000
(51) Int. Cl.: C07C 29/149, B01J 23/80

(54) **KATALYSATOR UND VERFAHREN ZUR HYDRIERUNG VON CARBONYLVERBINDUNGEN**
CATALYST AND METHOD FOR HYDROGENATING CARBONYL COMPOUNDS
CATALYSEUR ET PROCEDE D'HYDROGENATION DE COMPOSES CARBONYLE

(30) Priorität: 08.09.1999 DE 19942895
(43) Veröffentlichungstag der Anmeldung: 03.07.2002
(73) Patentinhaber: BASF AKTIENGESELLSCHAFT, 67056 Ludwigshafen (DE)
(72) Erfinder: HUBER, Sylvia, 64673 Zwingenberg (DE); SPRAGUE, Michael, Jolyon, 68161 Mannheim (DE); BREITSCHEIDEL, Boris, 67117 Limburgerhof (DE); WULFF-DÖRING, Joachim, 67227 Frankenthal (DE); HESSE, Michael, 67549 Worms (DE); PINKOS, Rolf, 67098 Bad Dürkheim (DE); LIANG, Shelue, 67071 Ludwigshafen (DE); KUMBERGER, Otto, Tsim Sha Tsui East Knowloon, Hong kong (CN); WALTER, Marc, 67227 Frankenthal (DE)
(74) Vertreter: Isenbruck, Günter, Dr.
(86) Internationale Anmeldenummer: PCT/EP2000/008195
(87) Internationale Veröffentlichungsnummer: WO 2001/017934

(56) Entgegenhaltungen:
- EP-A- 0 217 513
- EP-A- 0 296 734
- EP-A- 0 523 818
- FR-A- 2 352 588
- GB-A- 1 281 112
- US-A- 5 334 779

## Beschreibung

Die vorliegende Erfindung betrifft ein Verfahren zur Hydrierung von organischen Verbindungen, die mindestens eine Carbonylgruppe aufweisen, unter Verwendung eines Katalysators, der sich unter anderem dadurch auszeichnet, daß bei seiner Herstellung Kupferpulver oder Zement zugegeben werden.

Die katalytische Hydrierung von Carbonylverbindungen wie beispielsweise Carbonsäuren oder Carbonsäureestem nimmt in den Produktionssträngen der chemischen Grundstoffindustrie eine bedeutende Stellung ein.

Die katalytische Hydrierung von Carbonylverbindungen wie z. B. Carbonsäureestem wird in technischen Verfahren fast ausschließlich in Festbettreaktoren durchgeführt. Als Festbettkatalysatoren werden, neben Katalysatoren vom Raney-Typ, vor allem geträgerte Katalysatoren, beispielsweise Kupfer-, Nickel- oder Edelmetall-Katalysatoren verwendet.

Die US 3,923,694 beschreibt beispielsweise einen Katalysator vom Typ Kupferoxid / Zinkoxid / Aluminiumoxid. Der Nachteil dieses Katalysators besteht darin, daß er während der Reaktion mechanisch nicht ausreichend stabil ist und daher relativ schnell zerfällt. Daraus resultiert ein Aktivitätsverlust und ein Aufbau von Differenzdruck über den Reaktor durch die zerfallenden Katalysator-Formkörper. In der Folge muß die Anlage vorzeitig abgestellt werden.

Die DE 198 09 418.3 beschreibt ein Verfahren zur katalytischen Hydrierung einer Carbonylverbindung in Gegenwart eines Katalysators, der einen Träger, der vornehmlich Titandioxid enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, wobei die Kupferoberfläche maximal 10 m²/g beträgt. Bevorzugte Trägermaterialien sind Mischungen aus Titandioxid mit Aluminiumoxid oder Zirkonoxid oder Aluminiumoxid und Zirkonoxid. In einer bevorzugten Ausführungsform wird das Katalysatormaterial unter Zusatz von metallischem Kupferpulver verformt.

Die DE-A 195 05 347 beschreibt ganz allgemein ein Verfahren zur Herstellung von Katalysatortabletten mit hoher mechanischer Festigkeit, wobei dem zu tablettierenden Material ein Metallpulver oder ein Pulver einer Metall-Legierung zugegeben wird. Unter anderem wird als Metallpulver Aluminiumpulver oder Kupferpulver zugegeben. Bei der Zugabe von Aluminiumpulver wird bei einem Kupferoxid / Zinkoxid / Aluminiumoxid-Katalysator allerdings ein Formkörper erhalten, der eine schlechtere Seitendruckfestigkeit aufweist, als ein Formkörper, der ohne Zusatz von Aluminiumpulver hergestellt wurde, und der erfindungsgemäße Formkörper zeigte bei seiner Verwendung als Katalysator eine schlechtere Konvertierungsaktivität als Katalysatoren, die ohne Zusatz von Aluminiumpulver hergestellt wurden. Ebenfalls offenbart ist dort ein Hydrierkatalysator aus NiO, ZrO₂, MoO₃ und CuO, dem bei der Herstellung unter anderem Cu-Pulver zugemischt wurde. Über die Selektivität oder die Aktivität sind in dieser Schrift jedoch keine Angaben gemacht.

Die DD 256 515 beschreibt ein Verfahren zur Herstellung von Alkoholen aus Synthesegas, wobei Katalysatoren auf der Basis von Cu / Al / Zn eingesetzt werden, die durch gemeinsame Vermahlung und Verpillung mit metallischem Kupferpulver gewonnen werden. Das Hauptaugenmerk liegt bei dem beschriebenen Verfahren auf der Herstellung von Gemischen aus C₁- bis C₅-Alkoholen, wobei eine Verfahrensführung gewählt wird, in dem der Reaktionsreaktor im oberen Schichtdrittel einen Katalysator enthält, der einen höheren Anteil an Kupferpulver aufweist, und im unteren Drittel einen Katalysator enthält, der einen geringeren Anteil an Kupferpulver aufweist.

Eine Aufgabe der vorliegenden Erfindung war es, ein Verfahren und einen Katalysator bereitzustellen, die die Nachteile des Standes der Technik nicht aufweisen und Verfahren zur katalytischen Hydrierung von Carbonylverbindungen sowie Katalysatoren bereitzustellen, wobei die Katalysatoren sowohl hohe mechanische Stabilität als auch hohe Hydrieraktivität aufweisen.

Es wurde gefunden, daß eine Ausführungsform der Tablettierung, bei der einem getrockneten oxidischen Material, das Trägermaterial und Aktivkomponente aufweist und Kupferoxid, Zinkoxid und Aluminiumoxid umfaßt, metallisches Kupferpulver oder Zementpulver oder ein Gemisch davon beigemischt wird, sowohl zu hohen Aktivitäten und Selektivitäten sowie zu einer hohen Stabilität des Formkörpers, der als Katalysator eingesetzt wird, führt.

Demgemäß betrifft die vorliegende Erfindung ein Verfahren zur Hydrierung einer mindestens eine Carbonylgruppe aufweisenden organischen Verbindung, bei dem die organische Verbindung in Anwesenheit von Wasserstoff mit einem Formkörper in Kontakt gebracht wird, der herstellbar ist gemäß einem Verfahren, in dem
(i) ein getrocknetes und calciniertes oxidisches Material, das Trägermaterial und Aktivkomponente aufweist, umfassend Kupferoxid, Zinkoxid und Aluminiumoxid, bereitgestellt wird,
(ii) dem oxidischen Material pulverförmiges metallisches Kupfer oder pulverförmiger Zement oder ein Gemisch davon zugegeben wird, wobei Kupferpulver und Zementpulver mit einer Korngrößenverteilung verwendet werden, bei der mindestens 45% der Kupfer- oder Zementpartikel Korngrößer im Bereich 10 bis 100 µm besitzen, und
(iii) das aus (ii) resultierende Gemisch zu einem Formkörper verformt wird.

In bevorzugten Ausführungsformen werden die erfindungsgemäßen Formkörper als Voll,- Tränk-, Schalen- und Fällkatalysatoren eingesetzt.

Als Trägermaterial wird im erfindungsgemäßen Katalysator bevorzugt ein Gemisch aus Aluminiumoxid, Zinkoxid und gegebenenfalls Zink-Aluminium-Spinell eingesetzt. Hinsichtlich der Herstellung des Trägermaterials existieren keine besonderen Beschränkungen. In einer bevorzugten Ausführungsform des erfindungsgemäßen Verfahrens wird eine wäßrige Lösung, die Zinknitrat und .Aluminiumnitrat enthält, mit Soda zur Reaktion gebracht und die entstehende Suspension filtriert und getrocknet, besonders bevorzugt in einem weiteren Schritt noch calciniert.

Der in dem erfindungsgemäßen Verfahren verwendete Katalysator zeichnet sich dadurch aus, daß die Aktivkomponente Kupfer und die Aktivkomponente Zink auf das verwendete Trägermaterial aufgebracht wird, wobei bzgl. der Aufbringungsmethode keinerlei Beschränkungen existieren.

Insbesondere kommen folgende Aufbringungsmethoden in Betracht:
A) Aufbringung einer Kupfersatzlösung und einer Zinksalzlösung oder einer Lösung, enthaltend Kupfer- und Zinksalz, in einer oder mehreren Tränkstufen auf den vorgefertigten Träger. Der Träger wird im Anschluß an die Tränkung getrocknet und gegebenenfalls calciniert.
   A1) Die Tränkung kann nach der sogenannten "incipient wetness"-Methode erfolgen, bei der der Träger entsprechend seiner Wasseraufnahmekapazität maximal bis zur Sättigung mit der Tränklösung befeuchtet wird. Die Tränkung kann aber auch in überstehender Lösung erfolgen.
   A2) Bei mehrstufigen Tränkverfahren ist es zweckmäßig, zwischen einzelnen Tränkschritten zu trocknen und gegebenenfalls zu calcinieren. Die mehrstufige Tränkung ist vorteilhaft besonders dann anzuwenden, wenn der Träger mit einer größeren Kupfermenge und/oder einer größeren Zinkmenge beaufschlagt werden soll.
   A3) Bevorzugt wird das anorganische Trägermaterial bei der Tränkung als vorgeformte Masse eingesetzt, beispielsweise als Pulver, Kugeln, Stränge oder Tabletten. Besonders bevorzugt wird der Einsatz als Pulver.
B) Fällung einer Kupfersalzlösung und einer Zinksalzlösung oder einer Lösung, enthaltend Kupfer- und Zinksalz, auf den vorgefertigten Träger. Dieser liegt in einer besonders bevorzugten Ausführungsform als Pulver in einer wäßrigen Suspension vor.
   B1) In einer Ausführungsform (I) wird eine Kupfersalzlösung und eine Zinksalzlösung oder ein Lösung, enthaltend Kupfer- und Zinksalz, bevorzugt mit Sodalösung, gefällt. Als Vorlage wird eine wäßrige Suspension des Trägermaterials verwendet.
   B2) In einer weiteren Ausführungsform (II) kann der Fällkatalysator in einem Mehr-Stufen-Prozeß hergestellt werden. Dabei wird in einer ersten Stufe ein Pulver gemäß den Angaben aus A3) hergestellt und getrocknet. Dieses Pulver wird in eine wäßrige Suspension überführt und als Vorlage äquivalent zu der in Ausführungsform (I) beschriebenen Verfahrensführung eingesetzt.

Ausgefällte Niederschläge, die aus A) oder B) resultieren, werden in üblicher Weise filtriert und vorzugsweise alkalifrei gewaschen, wie dies beispielsweise in der DE 198 09 418.3 beschrieben ist.

Sowohl die Endprodukte aus A) als auch die aus B) werden bei Temperaturen von 50 bis 150 °C, vorzugsweise bei 120 °C getrocknet und im Anschluß ggf. vorzugsweise 2 Stunden bei im allgemeinen 200 bis 600 °C, insbesondere bei 300 bis 500 °C calciniert.

Als Ausgangssubstanzen für A) und/oder B) können prinzipiell alle in den bei der Aufbringung verwendeten Lösungsmitteln löslichen Cu(I) und/oder Cu(II)-Salze, wie beispielsweise Nitrate, Carbonate, Acetate, Oxalate oder Ammonium-Komplexe, und analoge Zinksalze verwendet werden. Besonders bevorzugt für Verfahren gemäß A) und B) wird Kupfernitrat eingesetzt.

In dem erfindungsgemäßen Verfahren wird das oben beschriebene getrocknete und gegebenenfalls calcinierte Pulver bevorzugt zu Tabletten, Ringen, Ringtabletten, Extrudaten, Wabenkörpern oder ähnlichen Formkörpem verarbeitet. Hierfür sind sämtliche aus dem Stand der Technik geeigneten Verfahren denkbar.

Die Zusammensetzung des oxidischen Material ist im allgemeinen so beschaffen, daß der Anteil an Kupferoxid im Bereich von 40 bis 90 Gew.-%, der Anteil an Zinkoxid im Bereich von 10 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

In einer bevorzugten Ausführungsform betrifft die vorliegende Erfindung daher ein Verfahren, wie oben beschrieben, das dadurch gekennzeichnet ist, daß das oxidische Material
(a) Kupferoxid mit einem Anteil im Bereich von 60 ≤ x ≤ 80, vorzugsweise 65 ≤ x ≤ 75 Gew.-%,
(b) Zinkoxid mit einem Anteil im Bereich von 15 ≤ y ≤ 35, vorzugsweise 20 ≤ y ≤ 30 Gew.-% und
(c) Aluminiumoxid mit einem Anteil im Bereich von 2 ≤ z ≤ 20, bevorzugt 3 ≤ z ≤ 7 Gew.-%,
jeweils bezogen auf das Gesamtgewicht des oxidischen Materials nach Calcinierung, wobei gilt: 80 ≤ x + y + z ≤ 100, insbesondere 95 ≤ x + y + z ≤ 100, wobei Zement nicht dem oxidischen Material im obigen Sinne zugerechnet wird, umfaßt.

Das erfindungsgemäße Verfahren und die erfindungsgemäßen Katalysatoren zeichnen sich dadurch aus, daß als Additiv vor der Formgebung dem oxidischen Material pulverförmiges Kupfer oder pulverförmiger Zement oder ein Gemisch davon zugesetzt wird.

Im allgemeinen wird dem oxidischen Material pulverförmiges Kupfer oder pulverförmiger Zement oder ein Gemisch davon im Bereich von 1 bis 40 Gew.-%, bevorzugt im Bereich von 2 bis 20 Gew.-% und besonders bevorzugt im Bereich von 5 bis 10 Gew.-%, jeweils bezogen auf das Gesamtgewicht des oxidischen Materials, enthält.

Die vorliegende Erfindung betrifft daher auch ein Verfahren, wie oben beschrieben, das dadurch gekennzeichnet ist, daß das pulverförmige metallische Kupfer oder der pulverförmige Zement oder das Gemisch davon in einem Anteil im Bereich von 1 bis 40 Gew.-%, bezogen auf das Gesamtgewicht des oxidischen Materials, zugegeben wird.

Die Korngröße des Kupferpulvers oder des Zementpulvers liegt im allgemeinen im Bereich von 0,1 bis 1000 µm, bevorzugt im Bereich von 0,5 bis 500 µm und besonders bevorzugt im Bereich von 1 bis 300 µm.

Es werden Kupferpulver und Zementpulver mit einer Korngrößenverteilung verwendet, bei der mindestens 45%, bevorzugt mindestens 70%, besonders bevorzugt mindestens 90% der Kupfer- oder Zementpartikel Korngrößen im Bereich 10 bis 100 µm besitzen.

Die Korngrößen werden mit einem Partikelgrößenmeßgerät vom Typ "HELOS 12KA/LA" der Firma SYMPATEC bestimmt. Das SYMPATEC HELOS-System basiert auf dem optischen Prinzip der Laserbeugung zur schnellen Analyse von Partikelgrößenverteilungen in Suspensionen, Emulsionen, Aerosolen und Sprays. Das Meßsystem HELOS besteht aus einer optischen Anordnung, bei der Laser, Strahlaufweiter, Meßstelle, Sammellinse und Multielement-Fotodetektor nacheinander in der optischen Achse angeordnet sind. Die im Strahlengang nachgeordnete Sammellinse bündelt die durch die Partikel entstehenden Fraunhoferschen Beugungsspektren und bildet sie auf den im Brennpunkt zentrisch angeordneten Mulitelement-Fotodetektor ab. In Abhängigkeit von der vorliegenden Partikelgrößenverteilung entsteht eine radialsymmetrische Intensitätsverteilung, deren Energiedichte, mit der Entfernung vom Zentrum abnimmt und deren Verlauf von der Anzahl und der Größe der im Meßvolumen erfaßten Partikel bestimmt wird. Mit dem aus 31 halbkreisförmigen Ringen bestehenden Multielement-Detektor wird die Intensitätsverteilung aufgenommen, in spannungsproportionale Werte umgeformt, in einem nachgeschalteten Rechner zwischengespeichert und zur weiteren Auswertung übernommen. Aus den gemessenen Intensitäten läßt sich durch Lösen eines linearen Gleichungssystems die zugehörige Partikelgrößenverteilung errechnen.

Die Oberfläche des Kupferpulvers oder Zementpulvers, bestimmt nach der BET-Methode, liegt im allgemeinen im Bereich von 0,01 bis 20 m²/g, bevorzugt im Bereich von 0,05 bis 10 m²/g, besonders bevorzugt im Bereich von 0,1 bis 0,5 m²/g.

Daher betrifft die vorliegende Erfindung auch ein Verfahren, wie oben beschrieben, das dadurch gekennzeichnet ist, daß die Korngröße des pulverförmigen Kupfers und des pulverförmigen Zements im Bereich von 0,1 bis 1000 µm und die BET-Oberfläche im Bereich 0,01 bis 20 m²/g liegt.

Als Zement wird vorzugsweise ein Tonerdezement eingesetzt. Besonders bevorzugt besteht der Tonerdezement im wesentlichen aus Aluminiumoxid und Calciumoxid, und besonders bevorzugt besteht er aus ungefähr 75 bis 85 Gew.-% Aluminiumoxid und ungefähr 15 bis 25 Gew.-% Calciumoxid. Ferner kann ein Zement auf Basis Magnesiumoxid/Aluminiumoxid, Calciumoxid/Siliciumoxid und Calciumoxid/Aluminiumoxid/Eisenoxid verwendet werden

Insbesondere kann das oxidische Material in einem Anteil von höchstens 10 Gew.-%, bevorzugt höchstens 5 Gew.-%, bezogen auf das Gesamtgewicht des oxidischen Materials, mindestens eine weitere Komponente aufweisen, die ausgewählt wird aus der Gruppe bestehend aus den Elementen Re, Fe, Ru, Co, Rh, Ir, Ni, Pd und Pt.

In einer weiter bevorzugten Ausführungsform des erfindungsgemäßen Verfahrens wird dem oxidischen Material vor dem Verformen zum Formkörper zusätzlich zu dem Kupferpulver oder dem Zementpulver oder dem Gemisch davon Graphit zugesetzt. Vorzugsweise wird soviel Graphit zugegeben, daß die Verformung zu einem Formkörper besser durchgeführt werden kann. In einer bevorzugten Ausführungsform werden 0,5 bis 5 Gew.-% Graphit, bezogen auf das Gesamtgewicht des oxidischen Materials, zugegeben. Dabei ist es gleichgültig, ob Graphit dem oxidischen Material vor oder nach oder gleichzeitig mit dem Kupferpulver oder dem Zementpulver oder dem Gemisch davon zugesetzt wird.

Demgemäß betrifft die vorliegende Erfindung auch ein Verfahren, wie oben beschrieben, das dadurch gekennzeichnet, ist, daß dem oxidischen Material oder dem aus (ii) resultierendem Gemisch Graphit in einem Anteil im Bereich von 0,5 bis 5 Gew.-%, bezogen auf das Gesamtgewicht des oxidischen Materials, zugegeben wird.

In einer bevorzugten Ausführungsform betrifft die vorliegende Erfindung daher auch einen Formkörper, umfassend
ein oxidisches Material, das
(a) Kupferoxid mit einem Anteil im Bereich von 60 ≤ x ≤ 80, vorzugsweise 65 ≤ x ≤ 75 Gew.-%,
(b) Zinkoxid mit einem Anteil im Bereich von 15 ≤ y ≤ 35, vorzugsweise 20 ≤ y ≤ 30 Gew.-% und
(c) Aluminiumoxid mit einem Anteil im Bereich von 2 ≤ z ≤ 20, bevorzugt 3 bis 7 Gew.-%,
jeweils bezogen auf das Gesamtgewicht des oxidischen Materials nach Calcinierung, wobei gilt: 80 ≤ x + y + z ≤ 100, insbesondere 95 ≤ x + y+ z ≤ 100 umfaßt,
metallisches Kupferpulver oder Zementpulver oder ein Gemisch davon mit einem Anteil im Bereich von 1 bis 40 Gew.-%, bezogen auf das Gesamtgewicht des oxidischen Materials, und
Graphit mit einem Anteil von 0,5 bis 5 Gew.-%, bezogen auf das Gesamtgewicht des oxidischen Materials,
wobei die Summe der Anteile aus oxidischem Material, metallischem Kupferpulver oder Zementpulver oder einem Gemisch davon und Graphit mindestens 95 Gew.-% des Formkörpers ergeben.

Nach Zugabe des Kupferpulvers oder des Zementpulvers oder des Gemischs davon und gegebenenfalls Graphit zu dem oxidischen Material wird der im Anschluß an die Verformung erhaltene Formkörper gegebenenfalls mindestens einmal calciniert über eine Zeit von im allgemeinen 0,5 bis 10 h, bevorzugt 0,5 bis 2 Stunden. Die Temperatur bei diesem mindestens einen Calcinierschritt liegt im allgemeinen im Bereich von 200 bis 600 °C, bevorzugt im Bereich von 250 bis 500 °C und besonders bevorzugt im Bereich von 300 bis 400 °C.

Im Falle der Formgebung mit Zementpulver kann es vorteilhaft sein, den vor der Calcinierung erhaltenen Formkörper mit Wasser zu befeuchten und anschließend zu trocknen.

Bei Einsatz als Katalysator in der oxidischen Form wird der Formkörper vor Beschickung mit der Hydrierlösung mit reduzierenden Gasen, beispielsweise Wasserstoff, vorzugsweise Wasserstoff-Inertgasgemischen, insbesondere Wasserstoff/Stickstoffgemischen bei Temperaturen im Bereich von 100 bis 500 °C, bevorzugt im Bereich von 150 bis 350 °C und insbesondere im Bereich von 180 bis 200 °C vorreduziert. Bevorzugt wird dabei ein Gemisch mit einem Wasserstoffanteil im Bereich von 1 bis 100 Vol.-%, besonders bevorzugt im Bereich von 1 bis 50 Vol.-% verwendet.

In einer bevorzugten Ausführungsform wird der Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Katalysator vorab im Reduktionsofen aktiviert worden, wird er in den Reaktor eingebaut und direkt unter Wasserstoffdruck mit der Hydrierlösung beschickt.

Bevorzugtes Einsatzgebiet der nach dem erfindungsgemäßen Verfahren hergestellten Formkörper ist die Hydrierung von Carbonylgruppen aufweisenden organischen Verbindungen im Festbett. Andere Ausführungsformen wie beispielsweise die Wirbelreaktion mit in auf- und abwirbelnder Bewegung befindlichem Katalysatormaterial ist jedoch ebenfalls möglich. Die Hydrierung kann in der Gasphase oder in der Flüssigphase durchgeführt werden. Vorzugsweise wird die Hydrierung in flüssiger Phase durchgeführt, beispielsweise in Riesel- oder Sumpffahrweise.

Bei Arbeiten in Rieselfahrweise läßt man das flüssige, die zu hydrierende Carbonylverbindung enthaltende Edukt in dem Reaktor, der unter Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In einer Ausführungsform wird die zu hydrierende Lösung im geraden Durchgang über die Katalysatorschüttung gepumpt. In einer anderen Ausführungsform des erfindungsgemäßen Verfahrens wird ein Teil des Produkts nach Durchgang durch den Reaktor als Produktstrom kontinuierlich abgezogen und ggf. durch einen zweiten Reaktor, wie oben definiert, geleitet. Der andere Teil des Produkts wird zusammen mit frischem, die Carbonylverbindung enthaltendem Edukt dem Reaktor crneut zugeführt. Diese Verfahrensweise wird im folgenden als Kreislauffahrweise bezeichnet.

Wird als Ausführungsform des erfindungsgemäßen Verfahrens die Rieselfahrweise gewählt, ist hierbei die Kreislauffahrweise bevorzugt. Weiter bevorzugt wird in Kreislauffahrweise unter Verwendung eines Haupt- und Nachreaktors gearbeitet.

Das erfindungsgemäße Verfahren eignet sich zur Hydrierung von Carbonylverbindungen wie z. B. Aldehyden und Ketonen, Carbonsäuren, Carbonsäureestern oder Carbonsäureanhydriden zu den entsprechenden Alkoholen, wobei aliphatische und cycloaliphatische gesättigte und ungesättigte Carbonylverbindungen bervorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkoholen hydriert. Der Begriff "Carbonylverbindungen", wie er im Rahmen der Erfindung verwendet wird, umfaßt alle Verbindungen, die eine C=O-Gruppe aufweisen, einschließlich Carbonsäuren und deren Derivaten. Selbstverständlich können auch Gemische aus zwei oder mehr als zwei Carbonylverbindungen gemeinsam hydriert werden. Ferner kann auch die einzelne, zu hydrierende Carbonylverbindung mehr als eine Carbonylgruppe enthalten.

Bevorzugt wird das erfindungsgemäße Verfahren zur Hydrierung aliphatischer Aldehyde, Hydroxyaldehyde, Ketone, Säuren, Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind. Ferner können auch oligomere Verbindungen, die auch mehr als 30 Carbonylgruppen enthalten, hydriert werden.

Als Beispiel für aliphatische Aldehyde sind zu nennen:
Formaldehyd, Propionaldehyd, n-Butyraldehyd, iso-Butyraldehyd, Valeraldehyd, 2-Methylbutyraldehyd, 3-Methylbutyraldehyd (Isovaleraldehyd), 2,2-Dimethylpropionaldehyd (Pivalinaldehyd), Capronaldehyd, 2-Methylvaleraldehyd, 3-Methylvaleraldehyd, 4-Methylvaleraldehyd, 2-Ethylbutyraldehyd, 2,2-Dimethylbutyraldehyd, 3,3-Dimethylbutyraldehyd, Caprylaldehyd, Caprinaldehyd, Glutardialdehyd.

Neben den genannten kurzkettigen Aldehyden sind insbesondere auch langkettige aliphatische Aldehyde geeignet, wie sie beispielsweise durch Oxosynthese aus linearen α-Olefinen erhalten werden können.

Besonders bevorzugt sind Enalisierungsprodukte, wie z. B. 2-Ethylhexenal, 2-Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydroxy-2-ethylhexanal (Butylaldol), 3-Hydroxy-2-methylpentanal (Propienaldol), 2-Methylolpropanal, 2,2-Dimethylolpropanal, 3-Hydroxy-2-methylbutanal, 3-Hydroxypentanal, 2-Methylolbutanal, 2,2-Dimethylolbutanal, Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon, 2,3-Butandion, 2,4-Pentandion, 2,5-Hexandion und Methylvinylketon.

Darüber hinaus können Carbonsäuren und Derivate davon, vorzugsweise solche mit 1-20 C-Atomen umgesetzt werden. Insbesondere sind die folgenden zu nennen:
Carbonsäuren, wie z. B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Nitrobenzoesäure, Salicylsäure, p-Hydroxybenzoesäure, Anthranilsäure, p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;
Carbonsäureester, wie z. B. die C₁-C₁₀-Alkylester der oben genannten Carbonsäuren, insbesondere Methylformiat, Essigester, Buttersäurebutylester, Phthalsäure-, Isophthalsäure-, Terephthalsäure-, Adipinsäure-, Maleinsäuredialkylester wie z. B. die Dimethylester dieser Säuren, (Meth)acrylsäuremethylester, Butyrolacton, Caprolacton und Polycarbonsäureester, wie z. B. Polyacryl- und Polymethacrylsäureester und deren Copolymere und Polyester, wie z. B. Polymethylmethacrylat, Terephthalsäureester und andere technische Kunststoffe, wobei hier insbesondere Hydrogenolysen, also die Umsetzung von Estern zu den entsprechenden Säuren und Alkoholen, durchgeführt werden;
Fette;
Carbonsäureanhydride, wie z. B. die Anhydride der oben genannten Carbonsäuren, insbesondere Essigsäureanhydrid, Propionsäureanhydrid, Benzoesäureanhydrid und Maleinsäureanhydrid;
Carbonsäureamide, wie z. B. Formamid, Acetamid, Propionamid, Stearamid, Terephthalsäureamid.

Ferner können auch Hydroxycarbonsäuren, wie z. B. Milch-, Äpfel-, Wein- oder Zitronensäure, oder Aminosäuren, wie z. B. Glycin, Alanin, Prolin und Arginin, und Peptide umgesetzt werden.

Als besonders bevorzugte organische Verbindungen werden gesättigte oder ungesättigte Carbonsäuren, Carbonsäureester, Carbonsäureanhydride oder Lactone oder Gemische aus zwei oder mehr davon hydriert.

Demgemäß betrifft die vorliegende Erfindung auch ein Verfahren, wie oben beschrieben, das dadurch gekennzeichnet ist, daß die organische Verbindung eine Carbonsäure, ein Carbonsäureester, ein Carbonsäureanhydrid oder ein Lacton ist.

Beispiele dieser Verbindungen sind unter anderem Maleinsäure, Maleinsäureanhydrid, Bernsteinsäure, Bernsteinsäureanhydrid, Adipinsäure, 6-Hydroxycapronsäure, 2-Cyclododecylpropionsäure, die Ester der vorgenannten Säuren wie z. B. Methyl-, Ethyl-, Propyl- oder Butylester. Weitere Beispiele sind γ-Butyrolacton und Caprolacton.

In einer ganz besonders bevorzugten Ausführungsform betrifft die vorliegende Erfindung ein Verfahren, wie oben beschrieben, das dadurch gekennzeichnet ist, daß die organische Verbindung Adipinsäure oder ein Adipinsäureester ist.

Die zu hydrierende Carbonylverbindung kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF und NMP, besonders bevorzugt ist Wasser.

Die Hydrierung sowohl in Sumpf- als auch in Rieselfahrweise, wobei jeweils bevorzugt in Kreislauffahrweise gearbeitet wird, führt man im allgemeinen bei einer Temperatur im Bereich von 50 bis 350 °C, bevorzugt im Bereich von 70 bis 300 °C, besonders bevorzugt im Bereich von 100 bis 270 °C und einem Druck im Bereich von 3 bis 350 bar, bevorzugt im Bereich von 5 bis 330 bar, besonders bevorzugt im Bereich von 10 bis 300 bar durch.

In einer ganz besonders bevorzugten Ausführungsform werden die Katalysatoren gemäß der vorliegenden Erfindung in Verfahren zur Herstellung von Hexandiol und/oder Caprolacton eingesetzt, wie sie in DE 196 07 954, DE 196 07 955, DE 196 47 348 und DE 196 47 349 beschrieben sind.

Mit dem erfindungsgemäßen Verfahren werden hohe Umsätze und Selektivitäten erzielt. Gleichzeitig weisen die Katalysatoren eine hohe chemische und mechanische Stabilität auf. Von besonderer Bedeutung ist hierbei das . vorteilhafte Abriebsverhalten, das sich in niedrigen Abriebswerten äußert.

Die mechanische Stabilität von Festkörperkatalysatoren und speziell der Katalysatoren gemäß der vorliegenden Erfindung wird beschrieben durch die Parameter Abrieb und Seitendruckfestigkeit.

Die Seitendruckfestigkeit wurde im Rahmen der vorliegenden Anmeldung bestimmt mit einem Gerät des Typs "Z 2.5/T 919" der Firma Zwick (Ulm), der Abrieb nach ASTM Designation D 4058-81. Sowohl bei den reduzierten als auch bei den gebrauchten Katalysatoren wurden die Messungen unter Stickstoffatmosphäre durchgeführt, um eine Re-Oxidation der Katalysatoren zu vermeiden.

In den folgende Beispielen soll die Erfindung näher beschrieben werden.

### Beispiele

### Beispiel 1: Herstellung des Katalysators 1

### Herstellung des Trägers

Zu 649 g einer gut gerührten wäßrigen Lösung von Zinknitrat mit einem Zink-Gehalt von 14,5 Gew.-% wurden 450 g Al(NO₃)₃ * 9 H₂O zugegeben und das Gemisch mit Wasser auf ein Volumen von 1,25 1 gebracht, um das Aluminiumsalz in Lösung zu bringen (Lösung A). In einem separaten Gefäß wurden 474 g wasserfreies Soda in Wasser gelöst und die Lösung auf 2 1 mit Wasser aufgefüllt. (Lösung B).

Lösung A und Lösung B wurden auf 50 °C erhitzt und über getrennte Leitungen in ein Fällgefäß, das eine auf 50 °C erhitzte, gut gerührte Lösung von 20 g NaHCO₃ in 350 ml Wasser enthielt, geleitet. Hierbei wurde durch entsprechende Einstellung der Zufuhrgeschwindigkeiten der Lösungen A und B der pH-Wert innerhalb von ca. 3 Minuten auf 6,8 gebracht. Unter Konstanthaltung des pH-Wertes bei 6,8 und der Temperatur bei 50 °C wurde die gesamte Lösung A mit Soda zur Reaktion gebracht. Die so gebildete Suspension wurde anschließend 3 Stunden lang nachgerührt, wobei der pH-Wert durch gelegentliche Zugabe von verdünnter Salpetersäure bei 6,8 gehalten wurde. Die Suspension wurde filtriert und mit destilliertem Wasser so lange gewaschen, bis der Nitratgehalt des Waschwassers < 10 ppm betrug. Der Filterkuchen wurde 16 h lang bei 120 °C getrocknet und anschließend 1 h lang bei 425 °C calciniert.

### Herstellung des Katalysators

Ein Gemisch aus 432 g einer salpetersauren Kupfernitratlösung mit einem Kupfergehalt von 15,5 Gew.-% und 95 g einer salpetersauren Zinknitratlösung mit einem Zink-Gehalt von 14,5 Gew.-% wurde mit Wasser auf 500 ml verdünnt und auf 70 °C erwärmt. Unter Rühren wurden 25,1 g des oben beschriebenen pulverförmigen calcinierten Trägers während ca. 5 Minuten langsam zugegeben und die so erhaltene milchige Suspension 15 Minuten lang gerührt (Suspension C).

In einem separaten Gefäß wurden 474 g wasserfreies Soda in Wasser gelöst und die Lösung auf 2 l mit Wasser aufgefüllt und auf 70 °C erhitzt (Lösung D). Suspension C und Lösung D wurden über getrennte Leitungen in ein Fällgefäß, das mit einem Rührer versehen war und 350 ml auf 70 °C erhitztes Wasser enthielt, geleitet. Hierbei wurde durch entsprechende Einstellung der Zufuhrgeschwindigkeiten der Suspension C und Lösung D der pH-Wert auf 7,4 gebracht.

Unter Konstanthaltung des pH-Wertes bei 7,4 und der Temperatur bei 70 °C wurde die gesamte Suspension C mit Soda zur Reaktion gebracht. Die so gebildete Suspension wurde anschließend 2 Stunden lang nachgerührt, wobei der pH-Wert durch gelegentliche Zugabe von verdünnter Salpetersäure bzw. Sodalösung D bei 7,4 gehalten wurde. Die Suspension wurde filtriert und mit destilliertem Wasser so lange gewaschen, bis der Nitratgehalt des Waschwassers < 10 ppm betrug.

Der Filterkuchen wurde 16 h lang bei 120 °C getrocknet und anschließend 1 h lang bei 430 °C calciniert. Das so erhaltene braunschwarze Katalysatorpulver wurde mit 1,5 Gew.-% Graphit und 5 Gew.-% Kupferpulver (Typ FFL Nr. 10914 der Norddeutschen Affinerie mit einer BET-Oberfläche von 0,23 m²/g und einer Partikelgrößenverteilung, bei der 92% der Partikel im Größenbereich 10 bis 100 µm liegen) gemischt und zu Tabletten von 3 mm Durchmesser und 3 mm Höhe verpreßt. Die Tabletten wurden schließlich 1 h lang bei 330 °C calciniert.

Der so hergestellte Katalysator hat die chemische Zusammensetzung 66 % CuO / 24 % ZnO / 5 % Al₂O₃ / 5 % Cu. Die Seitendruckfestigkeit und der Abrieb im oxidischen und reduzierten Zustand sind in Tabelle 1 aufgeführt.

### Beispiel 2: Hydrierung von Adipinsäuredimethylester an Katalysator 1

Adipinsäuredimethylester wurde kontinuierlich in Rieselfahrweise mit Rückführung (Verhältnis Zulauf/Rückführung = 10/1) bei einer Belastung von 0,5 kg/(l*h), einem Druck von 240 bar und Reaktionstemperaturen von 200 °C bzw. 220 °C in einem senkrechten Rohrreaktor, der mit 200 ml Katalysator 1 gefüllt war, hydriert. Die Versuchsdauer betrug insgesamt 14 Tage. GC-analytisch wurden im Reaktoraustrag bei 200 °C bzw. 220°'C Esterumsätze von 99 % bzw. 100 %, Hexandiol-Anteile von 57 % bzw. 62 % und Methanol-Gehalte von 30 % bzw. 31 % detektiert. Nach Ausbau war der Katalysator noch voll erhalten und wies eine hohe mechanische Stabilität auf. Seitendruckfestigkeit und Abrieb sind in Tabelle 1 zusammengestellt. Die Versuchsergebnisse sind nochmals in Tabelle 2 zusammengefaßt.

### Beispiel 3: Herstellung von Katalysator 2

Die Herstellung von Katalysator 2 erfolgte analog der von Katalysastor 1 in Beispiel 1, jedoch wurden 10 % Kupferpulver der Firma Schlenck vom Typ Unicoat 2845 mit einer BET-Oberfläche von 2,34 m²/g und einer Partikelgrößenverteilung, bei der 77% der Partikel im Größenbereich 10 bis 100 µm liegen, zugesetzt und die Tabletten bei 400 °C calciniert.

Der so hergestellte Katalysator hat die chemische Zusammensetzung 63 % CuO / 22 % ZnO / 5 % Al₂O₃ / 10 % Cu. Die Seitendruckfestigkeit und der Abrieb im oxidischen und reduzierten Zustand sind in Tabelle 1 aufgeführt.

### Beispiel 4: Hydrierung von Adipinsäuredimethylester an Katalysator 2

Adipinsäuredimethylester wurde kontinuierlich in Rieselfahrweise mit Rückführung (Verhältnis Zulauf / Rückführung = 10/1) bei einer Belastung von 0,5 kg/(l*h), einem Druck von 240 bar und Reaktionstemperaturen von 200 °C bzw. 220 °C in einem senkrechten Rohrreaktor, der mit 200 ml Katalysator 2 gefüllt war, hydriert. Die Versuchsdauer betrug insgesamt 14 Tage. GC-analytisch wurden im Reaktoraustrag bei 200 °C bzw. 220 °C Esterumsätze von jeweils 98 %, Hexandiol-Anteile von 55 % bzw. 59 % und Methanol-Gehalte von 26 % bzw. 28 % detektiert. Nach Ausbau war der Katalysator noch voll erhalten und wies eine hohe mechanische Stabilität auf. Seitendruckfestigkeit und Abrieb sind in Tabelle 1 zusammengestellt. Die Versuchsergebnisse sind nochmals in Tabelle 2 zusammengefaßt.

### Beispiel 5:

Die Herstellung von Katalysator 3 erfolgte analog der von Katalysastor 2 in Beispiel 3, jedoch wurden 5 % Secarzement Typ 80 der Fa. Lafarge mit einer BET-Oberfläche von 7,5 m²/g und einer Partikelgrößenverteilung, bei der 49% der Partikel im Größenbereich 10 bis 100 µm liegen, zugesetzt. Die Tabletten wurden 6 h lang befeuchtet, an der Luft getrocknet und anschließend 2 h bei 400°C calciniert.

Der so hergestellte Katalysator hat die chemische Zusammensetzung 66 % CuO / 24 % ZnO / 5 % Al₂O₃ / 5 % Zement. Die Seitendruckfestigkeit und der Abrieb im oxidischen und reduzierten Zustand sind in Tabelle 1 aufgeführt.

### Beispiel 6: Hydrierung von Adipinsäuredimethylester an Katalysator 3

Adipinsäuredimethylester wurde kontinuierlich in Rieselfahrweise mit Rückführung (Verhältnis Zulauf / Rückführung = 10/1) bei einer Belastung von 0,5 kg/(l*h), einem Druck von 240 bar und Reaktionstemperaturen von 200 °C bzw. 220 °C in einem senkrechten Rohrreaktor, der mit 200 ml Katalysator 3 gefüllt war, hydriert. Die Versuchsdauer betrug insgesamt 14 Tage. GC-analytisch wurde im Reaktoraustrag bei 200 °C bzw. 220 °C Esterumsätze von 94 % bzw. 97 %, Hexandiol-Anteile von 50 % bzw. 57 % und Methanol-Gehalte von 26 % bzw. 28 % detektiert. Nach Ausbau war der Katalysator noch voll erhalten und wies eine hohe mechanische Stabilität auf. Seitendruckfestigkeit und Abrieb sind in Tabelle 1 zusammengestellt. Die Versuchsergebnisse sind nochmals in Tabelle 2 zusammengefaßt.

### Beispiel 7: Herstellung eines Vergleichskatalysators

Es wurde der Katalysator aus Beispiel 1 aus US 3 923 694 exakt nachgestellt. Der so hergestellte Katalysator hatte die chemische Zusammensetzung 70 % CuO / 25 % ZnO / 5 % Al₂O₃. Die Seitendruckfestigkeit und der Abrieb im oxidischen und reduzierten Zustand sind in Tabelle 1 aufgeführt.

### Beispiel 8: Hydrierung von Adipinsäuredimethylester am Vergleichskatalysator

Adipinsäuredimethylester wurde kontinuierlich in Rieselfahrweise mit Rückführung (Verhältnis Zulauf / Rückführung = 10/1) bei einer Belastung von 0,5 kg/(l*h), einem Druck von 240 bar und Reaktionstemperaturen von 200 °C bzw. 220 °C in einem senkrechten Rohrreaktor, der mit 200 ml des Vergleichskatalysators gefüllt war, hydriert. Die Versuchsdauer betrug insgesamt 14 Tage. GC-analytisch wurde im Reaktoraustrag bei 200 °C bzw. 220 °C Esterumsätze von 92% bzw. 96%, Hexandiol-Anteile von 48 % bzw. 58 % und Methanol-Gehalte von 25 % bzw, 28 % detektiert. Nach Ausbau war der Katalysator zwar noch voll erhalten, die mechanische Stabilität hatte aber deutlich nachgelassen. Seitendruckfestigkeit und Abrieb sind in Tabelle 1 zusammengestellt. Die Versuchsergebnisse sind in Tabelle 2 zusammengefaßt.

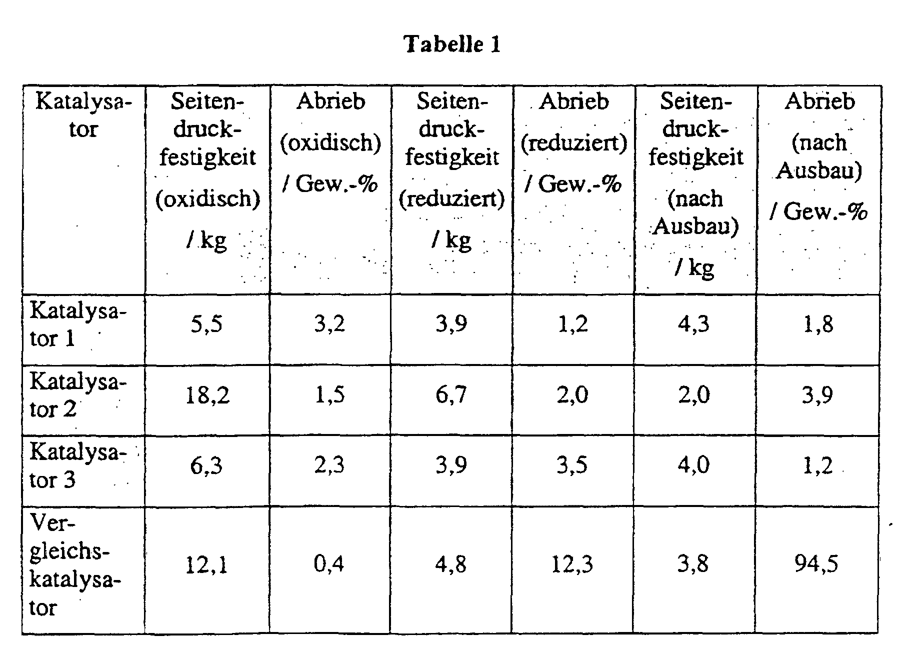

Die Daten in Tabelle 1 zeigen, daß die erfindungsgemäßen Katalysatoren 1 bis 3 im reduzierten Zustand und nach Ausbau eine deutlich höhere mechanische Stabilität, insbesondere wesentlich geringere Abriebswerte, zeigen als der Vergleichskatalysator.

Die Daten in der folgenden Tabelle 2 zeigen, daß die erfindungsgemäßen Katalysatoren signifikant höhere Hydrieraktivitäten, d. h. höhere Umsätze an Adipinsäuredimethylester bei 200 °C bzw. 220 °C aufweisen als der Vergleichskatalysator, sowie auch tendenziell höhrer Wertproduktselektivitäten, d. h. Gehalte an den Zielprodukten Hexandiol und Methanol im Austrag.

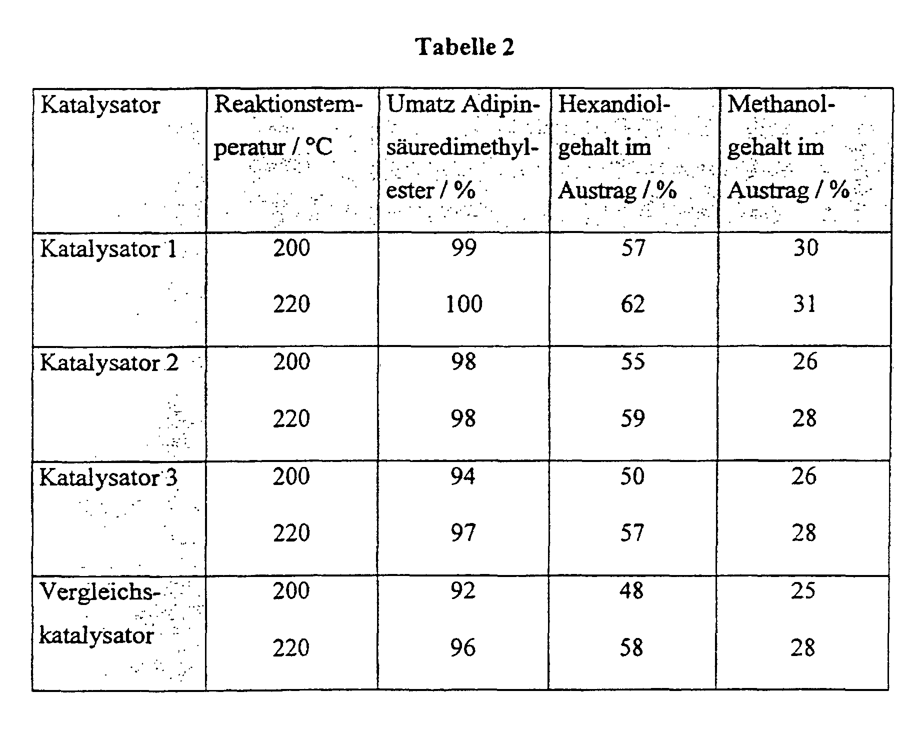

## Patentansprüche

1. Verfahren zur Hydrierung einer mindestens eine Carbonylgruppe aufweisenden organischen Verbindung, bei dem die organische Verbindung in Anwesenheit von Wasserstoff mit einem Formkörper in Kontakt gebracht wird, der herstellbar ist gemäß einem Verfahren, in dem
(i) ein getrocknetes und calciniertes oxidisches Material, das Trägermaterial und Aktivkomponente aufweist, umfassend Kupferoxid, Zinkoxid und Aluminiumoxid, bereitgestellt wird,
(ii) dem oxidischen Material pulverförmiges metallisches Kupfer oder pulverförmiger Zement oder ein Gemisch davon zugegeben wird, wobei Kupferpulver und Zementpulver mit einer Korngrößenverteilung verwendet werden, bei der mindestens 45% der Kupfer- oder Zementpartikel Korngrößen im Bereich 10 bis 100 µm besitzen, und
(iii) das aus (ii) resultierende Gemisch zu einem Formkörper verformt wird.

2. Verfahren nach Anspruch 1, **dadurch gekennzeichnet, dass** das oxidische Material
(a) Kupferoxid mit einem Anteil im Bereich von 60 ≤ x ≤ 80, vorzugsweise 65 ≤ x ≤ 75 Gew.-%,
(b) Zinkoxid mit einem Anteil im Bereich von 15 ≤ y ≤ 35, vorzugsweise 20 ≤ y ≤ 30 Gew.-% und
(c) Aluminiumoxid mit einem Anteil im Bereich von 2 ≤ z ≤ 20, bevorzugt 3 ≤ z ≤ 7 Gew.-%,
jeweils bezogen auf das Gesamtgewicht des oxidischen Materials nach Calcinierung, wobei gilt: 80 ≤ x + y + z ≤100, insbesondere 95 ≤ x + y + z ≤ 100, wobei Zement nicht dem oxidischen Material im obigen Sinne zugerechnet wird, umfasst.

3. Verfahren nach Anspruch 1 oder 2, **dadurch gekennzeichnet, dass** das pulverförmige metallische Kupfer oder der pulverförmige Zement oder das Gemisch davon in einem Anteil im Bereich von 1 bis 40 Gew.-%, bezogen auf das Gesamtgewicht des oxidischen Materials, zugegeben wird.

4. Verfahren nach einem der Ansprüche 1 bis 3, **dadurch gekennzeichnet, dass** die Komgröße des pulverförmigen Kupfers und des pulverförmigen Zements im Bereich von 0,1 bis 1000 µm liegt.

5. Verfahren nach einem der Ansprüche 1 bis 4, **dadurch gekennzeichnet, dass** dem oxidischen Material oder dem aus (ii) resultierendem Gemisch Graphit in einem Anteil im Bereich von 0,5 bis 5 Gew.-%, bezogen auf das Gesamtgewicht des oxidischen Materials, zugegeben wird.

6. Verfahren nach einem der Ansprüche 1 bis 5, **dadurch gekennzeichnet, dass** die organische Verbindung eine Carbonsäure, ein Carbonsäureester, ein Carbonsäureanhydrid oder ein Lacton ist.

7. Verfahren nach Anspruch 6, **dadurch gekennzeichnet, dass** die organische Verbindung Adipinsäure oder ein Adipinsäureester ist.

## Claims

1. A process for the hydrogenation of an organic compound containing at least one carbonyl group, which comprises bringing the organic compound in the presence of hydrogen into contact with a shaped body which can be produced by a process in which
(i) a dried and calcined oxidic material made up of support material and active component and comprising copper oxide, zinc oxide and aluminum oxide is made available,
(ii) pulverulent metallic copper or pulverulent cement or a mixture thereof is added to the oxidic material, with use being made of copper powder and cement powder having a particle size distribution in which at least 45% of the copper or cement particles have particle sizes in the range from 10 to 100 µm, and
(iii) the mixture resulting from (ii) is shaped to form a shaped body.

2. A process as claimed in claim 1, wherein the oxidic material comprises
(a) copper oxide in a proportion in the range 60 ≤ x ≤ 80% by weight, preferably 65 ≤ x ≤ 75% by weight,
(b) zinc oxide in a proportion in the range 15 ≤ y ≤ 35% by weight, preferably 20 ≤ y ≤ 30% by weight, and
(c) aluminum oxide in a proportion in the range 2 ≤ z ≤ 20% by weight, preferably 3 ≤ z ≤ 7% by weight,
in each case based on the total weight of the oxidic material after calcination, where 80 ≤ x + y + z ≤ 100, in particular 95 ≤ x + y + z ≤ 100, and cement is not included as part of the oxidic material in the above sense.

3. A process as claimed in claim 1 or 2, wherein the pulverulent metallic copper or the pulverulent cement or the mixture thereof is added in an amount in the range from 1 to 40% by weight, based on the total weight of the oxidic material.

4. A process as claimed in any of claims 1 to 3, wherein the particle size of the pulverulent copper and of the pulverulent cement is in the range from 0.1 to 1000 µm.

5. A process as claimed in any of claims 1 to 4, wherein graphite is added in an amount in the range from 0.5 to 5% by weight, based on the total weight of oxidic material, to the oxidic material or the mixture resulting from (ii).

6. A process as claimed in any of claims 1 to 5, wherein the organic compound is a carboxylic acid, a carboxylic ester, a carboxylic anhydride or a lactone.

7. A process as claimed in claim 6, wherein the organic compound is adipic acid or an ester of adipic acid.

## Revendications

1. Procédé d'hydrogénation d'un composé organique contenant au moins un groupe carbonyle, dans lequel le composé organique est mis en contact, en présence d'hydrogène, avec un corps moulé qui peut être préparé par un procédé dans lequel
(i) il est procuré un matériau oxydique séché et calciné, qui présente un matériau de support et un composant actif, comprenant de l'oxyde de cuivre, de l'oxyde de zinc et de l'oxyde d'aluminium,
(ii) on ajoute au matériau oxydique du cuivre métallique en poudre ou du ciment en poudre ou un mélange de ceux-ci, la poudre de cuivre et la poudre de ciment étant utilisées avec une distribution granulométrique dans laquelle au moins 45% des particules de cuivre ou de ciment possèdent des tailles de grains de l'ordre de 10 à 100 µm, et
(iii) !e mélange résultant de (ii) est façonné en un corps moulé.

2. Procédé selon la revendication 1, **caractérisé en ce que** le matériau oxydique comprend
a) de l'oxyde de cuivre avec une fraction de l'ordre de 60 ≤ x ≤ 80, de préférence de 65 ≤ x ≤ 75% en poids,
b) de l'oxyde de zinc avec une fraction de l'ordre de 15 ≤ y ≤ 35, de préférence de 20 ≤ y ≤ 30% en poids et
c) de l'oxyde d'aluminium avec une fraction de l'ordre de 2 ≤ z ≤ 20, de préférence de 3 ≤ z ≤ 7% en poids,
à chaque fois par rapport au poids total du matériau oxydique après calcination, avec 80 ≤ x + y + z ≤ 100, en particulier 95 ≤ x + y + z ≤ 100, le ciment n'étant pas compté dans le matériau oxydique au sens ci-dessus.

3. Procédé selon la revendication 1 ou 2, **caractérisé en ce que** le cuivre métallique en poudre ou le ciment en poudre ou le mélange de ceux-ci est ajouté en une fraction de l'ordre de 1 à 40% en poids, par rapport au poids total du matériau oxydique.

4. Procédé selon l'une quelconque des revendications 1 à 3, **caractérisé en ce que** la granulométrie du cuivre en poudre et du ciment en poudre est de 0,1 à 1000 µm.

5. Procédé selon l'une quelconque des revendications 1 à 4, **caractérisé en ce que** l'on ajoute au matériau oxydique ou au mélange résultant de (ii) du graphite, en une fraction de l'ordre de 0,5 à 5% en poids, par rapport au poids total du matériau oxydique.

6. Procédé selon l'une quelconque des revendications 1 à 5, **caractérisé en ce que** le composé organique est un acide carboxylique, un ester d'acide carboxylique, un anhydride d'acide carboxylique ou une lactone.

7. Procédé selon la revendication 6, **caractérisé en ce que** le composé organique est de l'acide adipique ou un ester d'acide adipique.
